# EUROPÄISCHE PATENTSCHRIFT

(11) **EP 1 196 592 B1**
(45) Veröffentlichungstag und Bekanntmachung des Hinweises auf die Patenterteilung: **23.11.2005**
(21) Anmeldenummer: 00949230.7
(22) Anmeldetag: 29.06.2000
(51) Int. Cl.: C12N 15/31, C12N 9/10, C12N 15/80, C12N 5/10

(54) **Expressionssystem zur Produktion von Proteinen in Pilzen**
Expression system for the production of proteins in fungi
Système d'expression pour la production des protéines dans des champignons

(30) Priorität: 22.07.1999 DE 19934408
(43) Veröffentlichungstag der Anmeldung: 17.04.2002
(73) Patentinhaber: Consortium für elektrochemische Industrie GmbH, 81379 München (DE)
(72) Erfinder: PFALLER, Rupert, D-80639 München (DE)
(74) Vertreter: Potten, Holger
(86) Internationale Anmeldenummer: PCT/EP2000/006091
(87) Internationale Veröffentlichungsnummer: WO 2001/007620

(56) Entgegenhaltungen:
- EP-A- 0 570 096
- WO-A-98/55628
- RASMUSSEN JACK B ET AL: "The PYR1 gene of the plant pathogenic fungus Colletotrichum graminicola: Selection by intraspecific complementation and sequence analysis." MOLECULAR & GENERAL GENETICS, Bd. 235, Nr. 1, 1992, Seiten 74-80, XP002151888 ISSN: 0026-8925

## Beschreibung

Die Erfindung betrifft ein Expressionssystem zur Produktion von Proteinen in Pilzen der Gattungen Trametes, Coriolus oder Polyporus.

Zur Proteinproduktion sind verschiedene prokaryontische und eukaryontische Expressionssysteme bekannt. Die Anmeldung DE-A-19814853 beschreibt ausführlich den diesbezüglichen Stand der Technik. DE-A-19814853 selbst offenbart ein Verfahren zur Transformation von filamentösen Pilzen aus den Gattungen Trametes und Polyporus, mit dem signifikant höhere Produktionsraten für ein jeweils exprimiertes Protein erzielt werden können. Die Anmeldung offenbart Expressionsvektoren, die genetische Regulationselemente für die Expression in filamentösen Pilzen der Klasse der Basidiomyceten enthalten. Sie erlauben bei Transformation filamentöser Pilze der Klasse der Basidiomyceten die Selektion positiver Transformanten aufgrund der Komplementation eines auxotrophen Gendefekts.

Der in DE-A-19814853 offenbarte Gendefekt betrifft das pyrG-Gen. Dieses Gen kodiert für die Orotidin-5'-Phosphat Decarboxylase. DE-A-19814853 offenbarte ferner Stämme mit einem Defekt im pyrG-Gen, die auf Minimalmedium nur in Gegenwart von Uridin wachsen können (Uridin Auxotrophie). Nach Transformation dieser Stämme mit DNS-Vektoren, die ein intaktes pyrG-Gen enthalten, wachsen die Uridin-auxotrophen Stämme wieder auf Minimalmedium ohne Uridin (Uridin Prototrophie).

Uridin-auxotrophe Stämme werden nach dem Stand der Technik (Boeke et al., Methods Enzymol. (1987) 154, 164 - 175) durch Behandlung mit der genotoxischen Substanz 5-Fluor-Orotsäure (FOA) isoliert. Bei der Behandlung mit FOA erzeugte Uridin-auxotrophe Stämme besitzen einen genetischen Defekt entweder im pyrG-Gen oder im pyrF-Gen. Das pyrF-Gen wird auch ura5-Gen genannt. Es kodiert für das Enzym Orotsäure-Phosphoribosyltransferase.

Auch Uridin-auxotrophe Basidiomycetenstämme mit einem Defekt im pyrF-Gen wären wertvolle Stämme für die Transformation zum Zweck der Proteinproduktion, wenn das intakte pyrF-Gen aus Basidiomyceten als Selektionsmarkergen für die effiziente Transformation zur Verfügung stände. PyrF-Gene sind aber bisher nur für Pilze aus der Klasse der Ascomyceten wie z. B. Podospora anserina (Gene 53 (1987), 201 - 209), Kluyveromyces lactis (unveröffentlicht, die DNS-Sequenz ist in der "Genbank" Datenbank unter der Zugangsnummer klj001358.gb_pl abgelegt) oder Yarrowia lipolytica (M. Sanchez et al., Yeast 11 (1995), 425 - 433) beschrieben worden. Dagegen sind keine pyrF-Gene von filamentösen Pilzen aus der Klasse der Basidiomyceten wie z.B. der Gattungen Trametes, Coriolus oder Polyporus bekannt.

Die Erfindung betrifft ein Expressionssystem umfaßend einen Wirtsstamm ausgewählt aus den filamentösen Pilzen aus der Klasse der Basidiomyceten mit einem genetischen Defekt im Stoffwechselmetabolismus aufgrund dessen der für das Wachstum essentielle Stoffwechselmetabolit Uridin nicht mehr synthetisiert werden kann und der Wirtsstamm auf Minimalmedien ohne Zusatz dieses Stoffwechselmetaboliten nicht mehr zum Wachstum befähigt ist sowie einen Expressionsvektor enthaltend ein Selektionsmarkergen, welches den auxotrophen Gendefekt des Wirtsstammes komplementiert, dadurch gekennzeichnet, daß der Wirtsstamm als genetischen Defekt im Stoffwechselmetabolismus einen Defekt im pyrF Gen besitzt, und das Selektionsmarkergen ein pyrF Gen aus einem Pilz der Klasse der Basidiomyceten ist.

Bevorzugt als Wirt für die Genexpression ist ein monokaryontischer Basidiomycet aus der Gattung Trametes, Coriolus oder Polyporus.

Insbesondere bevorzugt für die Genexpression ist ein Wirt der Art Trametes versicolor, der einen Defekt im pyrF Gen hat und für Uridin auxotroph ist.

Das pyrF Gen stammt bevorzugt aus einem Pilz der Gattung Agaricus, Coriolus, Polyporus, Pleurotus, Phanerochaete, Schizophyllum oder Trametes.

Insbesondere geeignet als Selektionsmarkergen für das erfindungsgemäße Expressionssystem ist das Orotsäure-Phosphoribosyltransferasegen (pyrF Gen) aus einem filamentösen Pilz der Klasse der Basidiomyceten Trametes versicolor.

Bevorzugt geeignet für die Expression des pyrF Gens sind die im Folgenden beschriebenen Expressionsvektoren.

Die Expressionsvektoren eignen sich insbesondere zur Expression von Genen die für Proteine kodieren in einem Wirtsorganismus der Gattung Trametes, Coriolus und Polyporus. Unter Genen die für Proteine kodieren sind im Sinne der Erfindung auch die von den Strukturgenen abgeleiteten cDNS-Gene der Proteine zu verstehen. Bei den Proteinen kann es sich um für den Wirtsorganismus heterologe Proteine oder um für den Wirtsorganismus homologe Proteine handeln.

Der Expressionsvektor enthält somit vorzugsweise auch mindestens ein Gen, das für ein zu exprimierendes Protein kodiert.

Besonders bevorzugt enthält der Expressionsvektor mindestens ein Gen, das für ein hydrolytisches Enzym z.B. aus der Gruppe der Cellulasen, Hemicellulasen und Lipasen oder aus der Gruppe der Oxidoreduktasen wie z.B. den Ligninperoxidasen, Manganperoxidasen, Laccasen, Cellobiose-Chinon-Oxidoreductase oder Cellobiose-Oxidase kodiert.

Insbesondere bevorzugt enthält der Expressionsvektor ein Gen für eine Laccase.

Bei dem Expressionsvektor kann es sich um ein DNS-Konstrukt handeln, das in das Genom des Wirtsorganismus integriert und zusammen mit diesem repliziert wird. Alternativ kann es sich bei dem Expressionsvektor um ein autonom replizierendes DNS-Konstrukt handeln, das nicht in das Wirtsgenom integriert, wie z.B. ein Plasmid, ein artifizielles Chromosom oder ein vergleichbares extrachromosomales genetisches Element.

Ein Expressionsvektor sollte vorzugsweise auch folgende genetische Elemente enthalten:

Einen Promotor, der die Expression eines proteinkodierenden Gens in dem Wirtsorganismus vermittelt. Es sollte sich dabei vorzugsweise um einen starken Promotor handeln, damit eine hohe Expressionsleistung gewährleistet werden kann. Der Promotor ist vorzugsweise funktionell mit dem 5'-Ende des zu exprimierenden Gens verknüpft. Der Promotor kann von dem zu exprimierenden Gen stammen oder es kann auch der Promotor eines fremden Gens verwendet werden.

Vorzugsweise geeignete Promotoren sind ausgewählt aus der Gruppe der in filamentösen Pilzen der Klasse der Basidiomyceten aktiven Promotoren wie z. B. der GAPDH-Promotor aus Trametes versicolor, Promotoren für Laccasegene aus Trametes versicolor oder Polyporus pinsitus, der Promotor für das Ornithin-Transcarbamoylasegen oder das GAPDH-Gen aus Coriolus hirsutus oder der GAPDH-Promotor aus Agaricus bisporus.

Besonders bevorzugt ist der GAPDH-Promoter aus Trametes versicolor. Dieser Promotor ist in DE-A-19814853 Beispiel 5 und DE-A-19814853, SEQ ID NO: 3, Base 1 - 1542 offenbart.

Ferner sollten vorzugsweise für den Wirtsorganismus passende Signale für die Transkriptionstermination und in Eukaryonten zusätzlich Signale für die Polyadenylierung in dem Expressionsvektor enthalten und funktionell mit dem 3'-Ende des zu exprimierenden Gens verknüpft sein. Solche Signale für die Transkriptionstermination und Polyadenylierung sind z.B. in SEQ ID NO:1, bp 1878 - 3448 gezeigt.

Als Transkriptionsterminator kann der Terminator des zu exprimierenden proteinkodierenden Gens verwendet werden oder aber der Terminator eines fremden Gens. Bevorzugt wird der Transkriptionsterminator aus dem Gen einer Laccase verwendet.

Die Expression der Proteine kann intrazellulär oder in Gegenwart einer funktionsfähigen Signalsequenz, zum Zweck der Sekretion, auch extrazellulär erfolgen.

Falls die Sekretion des exprimierten Proteins aus der Zelle erwünscht ist, enthält der Expressionsvektor vorzugsweise eine funktionstüchtige Signalsequenz 5' vor dem proteinkodierenden Gen. Darüberhinaus kann auch ein sogenannter Sekretionscarrier, funktionell mit dem 5'-Ende des proteinkodierenden Gens verknüpft, in dem Expressionsvektor enthalten sein.

Bei dem Sekretionscarrier kann es sich um das Gen für ein sekretiertes Protein oder um das Fragment eines Gens für ein sekretiertes Protein handeln. Der Sekretionscarrier kann mit dem zu sekretierenden Protein funktionell so verknüpft sein, daß ein Fusionsprotein aus Sekretionscarrier und dem zu sekretierenden Protein entsteht. In einer anderen Ausführung ist die Verknüpfung von Sekretionscarrier und dem zu sekretierenden Protein so gestaltet, daß der Sekretionscarrier von dem zu sekretierenden Protein getrennt werden kann. Dies kann beispielsweise bewerkstelligt werden durch Einfügung einer Erkennungssequenz für eine proteinspaltendes Enzym in die Verknüpfungsstelle zwischen Sekretionscarrier und zu sekretierendem Protein. Als Beispiel hierfür sei genannt die Lysin-Arginin Erkennungssequenz für die sogenannte KEX2-Protease und als Beispiel für einen Sekretionscarrier die Glucoamylase aus Aspergillus niger (Contreras et al., Bio/Technology (1991) 9, 378 - 381, Broekhuijsen et al., J. of Biotechnology (1993) 31, 135 - 145).

DNS-Sequenzen, die anders als Transkriptionsterminatoren am 3'-Ende des proteinkodierenden Gens an der Expression und Sekretion des exprimierten Gens beteiligt sind, können ebenfalls in dem Expressionsvektor enthalten sein. Ein Beispiel dafür liefert das Gen für die Laccase aus Neurospora crassa, dessen 3'-Ende die Sequenz für 13 Aminosäuren enthält, die während der Sekretion des Proteins entfernt werden und im reifen Protein nicht mehr enthalten sind (Germann et al., J. Biol. Chem. (1988) 263, 885 - 896).

Die Herstellung der Expressionsvektoren erfolgt mittels im Stand der Technik bekannter Verfahren. Verschiedene Möglichkeiten sind in den Beispielen dargelegt. Die dort beschriebenen Verehren lassen sich vom Fachmann auf beliebige andere Vektoren, Resistenzgene, Regulationselemente und Strukturgene anwenden.

Die Expressionsvektoren erlauben die Selektion positiver Transformanten aufgrund Komplementation eines auxotrophen Gendefekts im Wirtsorganismus bei Transformation von Pilzen ausgewählt aus den Gattungen Trametes, Coriolus und Polyporus.

Vorzugsweise wird die Expression des pyrF Gens aus dem Basidiomyceten Trametes versicolor vom Promotor und ggf. Terminator für das pyrF Gen aus Trametes versicolor reguliert.

Die Transformation des Wirtsstammes erfolgt nach Methoden, die dem Stand der Technik entsprechen. Zu diesen Methoden gehören die Transformation von Protoplasten nach der CaCl₂/PEG-Methode, die Transformation durch Elektroporation oder die biolistische Transformation durch Beschuß mit DNS-haltigen Mikroprojektilen. Diese Verfahren sind in Standardlehrbüchern beschrieben.

Beispielsweise wird das zu transformierende Gen in bekannter Art und Weise in einen Expressionsvektor kloniert und mittels der genannten Methoden in einen filamentösen Pilz ausgewählt aus den Gattungen Trametes, Coriolus und Polyporus eingebracht.

Das zu transformierende Gen kann aber auch in einen Expressionsvektor ohne Selektionsmarkergen kloniert werden und zusammen mit einem den auxotrophen Gendefekt des Wirtsstammes komplementierenden Vektor zur Erzeugung von Transformanten verwendet werden (Cotransformation).

Der für die Transformation zu verwendende Stamm ist ein Uridin-auxotropher filamentöser Pilz ausgewählt aus den Gattungen Trametes, Coriolus und Polyporus. Bei dem betreffenden Stamm aus der Klasse der Basidiomyceten kann es sich um einen monokaryontischen oder aber auch um einen dikaryontischen Stamm handeln. In einer bevorzugten Ausführung handelt es sich um einen Uridin-auxotrophen Stamm, der einen Defekt im pyrF Gen hat.

Besonders bevorzugt für die Transformation ist ein monokaryontischer, Uridin-auxotropher, pyrF defizienter Stamm aus der Art Trametes versicolor.

Die Selektion positiver Transformanten erfolgt beispielsweise, indem Protoplasten nach der Transformation mit Vektor DNS auf einem Medium ausgebracht werden, welchem zur osmotischen Stabilisierung der Protoplasten ein Zusatz wie z. B. Sorbitol, Mannitol oder Saccharose beigefügt ist und welches die Selektion von Transformanten mit dem komplementierenden pyrF- Gen erlaubt.

In einer bevorzugten Ausführung der Erfindung wird in einem homologen System der filamentöse Pilz Trametes versicolor mit dem Gen einer Laccase aus Trametes versicolor transformiert. Dadurch wird eine Steigerung der Expressionsrate für die besagte Laccase erzielt, die die nach dem Stand der Technik erzielbare Produktionsrate in der Fermentation von 0,1 g Laccase/l Kulturmedium signifikant verbessert.

Vorzugsweise verwendet man dazu den Promotor, der dem Laccasegen eigen ist oder den Promotor für ein stark exprimiertes Gen aus Trametes versicolor. Vorzugsweise verwendet man die Promotoren der Laccasegene I und III, deren Isolierung und Verwendung in DE-A-19814853 offenbart ist. Den Promotor eines weiteren stark exprimierten Gens stellt der GAPDH Promotor für die Glycerinaldehyd-3-Phosphatdehydrogenase aus Trametes versicolor dar.

Vorzugsweise verwendet man Selektionsmedien, auf denen nur solche Transformanten von Trametes versicolor wachsen können, die mit einem funktionell exprimierten Selektionsmarkergen für das pyrF-Gen transformiert worden waren. Bevorzugt handelt es sich um ein im 6. Beispiel beschriebenes Minimalmedium in Abwesenheit von Uridin, auf dem pyrF auxotrophe Stämme von Trametes versicolor nicht mehr wachsen können, bzw. erst nach Zusatz von Uridin wieder wachsen können.

Die erfolgreiche Anwendung eines Expressionsvektors enthaltend das pyrF Gen als Selektionssystem ist abhängig von der effizienten Expression des Selektionsmarkergens in Trametes Transformanten. Für eine effiziente Expression sind entsprechende Expressionssignale notwendig.

In Trametes versicolor bewirken Expressionssignale aus Basidiomyceten mit überraschenderweise erheblich höherer Effizienz eine funktionelle Expression als die anderweitig verfügbaren Expressionssignale aus Ascomyceten. Deshalb stehen bei den DNS Vektoren das pyrF Selektionsmarkergen vorzugsweise unter der Kontrolle von genetischen Regulationselementen aus Basidiomyceten, insbesondere bevorzugt von solchen ausgewählt aus den Gattungen Trametes, Coriolus und Polyporus.

Vorzugsweise steht das pyrF Gen unter der Kontrolle der ihm vorgeschalteten 5'-Promotorregion sowie der ihm nachgeschalteten 3'-Terminatorregion. Ein solches DNS-Fragment, bei dem das pyrF Gen aus Trametes versicolor unter Kontrolle der Expressionssignale des pyrF Gens aus Trametes versicolor steht, beschreibt SEQ ID NO:1.

Weiterhin kann das pyrF Gen unter der Kontrolle von Expressionssignalen aus Basidiomyceten stehen, die verschieden von denen des pyrF Gens sind. Zu den Expressionssignalen, die diese Funktion erfüllen, gehören GAPDH-Promotoren filamentöser Pilze aus der Klasse der Basidiomyceten, wie z.B. Coriolus hirsutus, Phanerochaete chrysosporium, Agaricus bisporus oder Trametes versicolor, der OCT-Promotor aus Coriolus hirsutus, der Promotor der Laccase I oder der Laccase III aus Trametes versicolor sowie der Terminator des GAPDH-Gens aus Agaricus bisporus oder die Terminatoren des Laccase I, bzw. Laccase III Gens aus Trametes versicolor.

Besonders bevorzugt ist ein Vektor, bei dem das pyrF Gen aus Trametes versicolor unter Kontrolle der Expressionssignale des GAPDH Gens aus Trametes versicolor steht. Ein solcher Vektor ist im 4. Beispiel beschrieben.

Insbesondere bevorzugt ist ein Vektor, bei dem das pyrF Gen aus Trametes versicolor unter Kontrolle der Expressionssignale des pyrF Gens aus Trametes versicolor steht. Ein solcher Vektor ist im 3. Beispiel beschrieben. Das pyrF Gen kann jedes Gen sein, das für ein Protein mit der enzymatischen Aktivität einer Orotsäure-Phosphoribosyltransferase kodiert.

Bevorzugt stammt das pyrF Gen aus einem filamentösen Pilz aus der Klasse der Basidiomyceten, wie z.B. Agaricus bisporus, Phanerochaete chrysosporium, Coriolus hirsutus, Polyporus pinsitus, Schizophyllum commune oder Trametes versicolor.

Besonders bevorzugt ist das pyrF Gen aus Trametes versicolor.

Die Erfindung betrifft auch ein Verfahren zur Herstellung eines Proteins welches dadurch gekennzeichnet ist, daß das erfindungsgemäße Expressionssystem enthaltend ein das Protein kodierendes Gen zur Proteinproduktion eingesetzt wird oder dass ein Mikroorganismus der einen Expressionsvektor umfassend eine DNS-Sequenz, die für ein Protein mit Enzymaktivität der Orotsäure-Phosphoribosyltransferase (pyrF-Aktivität)kodiert und eine DNS-Sequenz SEQ ID NO:1 ab Position 1133 bis einschließlich Position 1877 umfaßt oder eine DNS-Sequenz SEQ ID NO:2 ab Position 1 bis einschließlich Position 684 umfaßt oder eine DNS-Sequenz mit einer Sequenzhomologie größer als 70 % zu den genannten Bereichen der DNS-Sequenz SEQ ID NO:1, oder SEQ ID NO:2 umfasst enthaltend ein das Protein kodierendes Gen kultiviert wird und das Protein aus der Kultur gewonnen wird.

In der vorliegenden Erfindung beziehen sich alle erwähnten Homologiewerte auf Ergebnisse, die mit dem Computerprogramm "Wisconsin Package Version 9.1, Genetics Computer Group (GCG), Madison, Wisconsin" erhalten werden. Die Homologiebestimmung erfolgt durch eine Suche in der Datenbank mit dem Unterprogramm "blast" und den voreingestellten Werten (Trefferhäufigkeit 10,0). Die ähnlichsten Sequenzen werden dann mit dem Unterprogramm "gap" auf Homologie untersucht. Hierbei werden die voreingestellten Parameter "gap creation penalty 50" und "gap extension penalty 3" verwendet, um DNS-Sequenzen zu vergleichen. Um Aminosäuresequenzen zu vergleichen, werden die voreingestellten Parameter "gap weight 8" und "length weight 2" verwendet.

Die DNS Sequenz SEQ ID NO:1 von Position 1133 bis einschließlich Position 1225 und ab Position 1287 bis einschließlich Position 1877, bzw. die davon abgeleitete cDNS-Sequenz SEQ ID NO:2 von Position 1 bis einschließlich Position 684 kodiert für ein Protein mit pyrF-Aktivität.

Die DNS-Sequenz SEQ ID NO:1 ab Position 1226 bis einschließlich Position 1286 ist ein Intron, das nicht in Aminosäuresequenz translatiert wird.

Die DNS-Sequenz SEQ ID NO:1 gibt von Position 1 bis Position 1132 die DNS Sequenz für die Promotorregion zur Transkription des pyrF-Gens aus Tramtetes versicolor wieder. Diese Promotorsequenz läßt sich gegen beliebige andere Promotorsequenzen zur Transkription austauschen.

Die DNS-Sequenz läßt sich beispielsweise durch Klonierung aus dem Basidiomycetenstamm Trametes versicolor TV-1 (hinterlegt bei der DSMZ Deutschen Sammlung von Mikroorganismen und Zellkulturen GmbH, D-38124 Braunschweig unter der Nummer DSM 11523) erhalten. Dazu wird mittels an sich bekannter Methoden eine Genbank von Trametes versicolor TV-1 erstellt. Es kann sich dabei um eine cDNS- oder um eine genomische Genbank handeln.

Zur Isolierung der DNS-Sequenz in der Genbank werden DNS-Sonden verwendet, die pyrF-spezifische DNS-Sequenzen enthalten. Solche DNS-Sonden lassen sich beispielsweise mittels PCR-Reaktion unter Verwendung von DNS-Primern aus genomischer DNS von Trametes versicolor TV-1 gewinnen.

Als Primer werden degenerierte DNS Abschnitte einer Länge von vorzugsweise 23 bis 26 bp verwendet, deren Sequenz durch Sequenzvergleich bekannter pyrF-Gene festgelegt wird. Die als Primer geeigneten DNS-Abschnitte erhält man vorzugsweise durch Oligonukleotidsynthese der festgelegten DNS-Abschnitte. Die Isolation eines pyrF-Gens kann beispielsweise wie in den Beispielen 1 bis 3 beschrieben erfolgen.

Ein beispielsweise derart isoliertes pyrF-Gen läßt sich mittels dem Fachmann bekannten Techniken wie beispielsweise der site directed Mutagenese an jeweils gewünschter Position der Sequenz modifizieren.

Zur Expression der DNS wird diese in an sich bekannter Art und Weise in einem Expressionsvektor kloniert, dieser das pyrF-Gen enthaltende Expressionsvektor in einen Mikroorganismus eingebracht und in dem Mikroorganismus exprimiert.

Solche Herstellungsverfahren sind prinzipiell beispielsweise bekannt aus Eggert et al., Appl. Environ. Microbiol (1996) 62, 1151. - 1158, Martinez et al., Appl. Microbiol. Biotechnol. (1994) 41, 500 - 504, oder WO 93/08272.

Die folgenden Beispiele dienen der weiteren Erläuterung der Erfindung. Die in den Beispielen verwendeten Standardmethoden zur Behandlung von DNS oder RNS, wie die Behandlung mit Restriktionsendonukleasen, DNS Polymerasen, Reverser Transkriptase etc. sowie die Standardverfahren wie Transformation von Bakterien, Southern und Northern Analyse, DNS Sequenzierung, radioaktive Markierung, Screening und PCR-Technologie wurden, wenn nicht anders angegeben, durchgeführt wie vom Hersteller empfohlen oder wenn keine Herstelleranleitung vorhanden war entsprechend dem aus den Standardlehrbüchern bekannten Stand der Technik. dass ein Mikroorganismus der einen Expressionsvektor umfassend eine DNS-Sequenz, die für ein Protein mit Enzymaktivität der Orotsäure-Phosphoribosyltransferase (pyrF-Aktivität)kodiert und eine DNS-Sequenz SEQ ID NO:1 ab Positi-on 1133 bis einschließlich Position 1877 umfaßt oder eine DNS-Sequenz SEQ ID NO:2 ab Position 1 bis einschließlich Position 684 umfaßt oder eine DNS-Sequenz mit einer Sequenzhomologie größer als 70 % zu den genannten Bereichen der DNS-Sequenz SEQ ID NO:1, oder SEQ ID NO:2 umfasst enthaltend ein das Protein kodierendes Gen kultiviert wird und das Protein aus der Kultur gewonnen wird.

### 1. Beispiel

### Isolierung einer pyrF-spezifischen DNS Sonde

Eine DNS-Sonde zur Isolierung eines pyrF-Gens wurde durch PCR-Amplifikation aus *T. versicolor* genomischer DNS mit degenerierten Primern erzeugt. Die degenerierten Primer wurden anhand eines Sequenzvergleichs bekannter pyrF-Gene konstruiert. Gene für die Orotsäure Phosphoribosyltransferase (bezeichnet als pyrf Gene oder, in einer anderen Nomenklatur bezeichnet als ura5 Gene) wurden in den folgenden Gendatenbanken gesucht: a) swissprot, b) sptrembl, c) pir, d) embl, e) genbank, f) em_tags, g) gb_tagsEMBL. Ura5, bzw. pyrF Gene folgender Organismen wurden für den Sequenzvergleich ausgewählt: Yarrowia lipolytica, Saccharomyces cerevisiae, Escherichia coli, Rhizomucor circinelloides, Colletotrichum graminicola, Trichoderma reesei und Sordaria macrospora. Die Aminosäuresequenzen der genannten pyrF Gene wurden verglichen. Durch den Sequenzvergleich konnten drei Peptide mit einer Länge von 6 bis 9 Aminosäuren identifiziert werden, die in allen pyrF-Proteinen vollständig konserviert waren. Zwei dieser Peptide wurden unter Berücksichtigung degenerierter Codons in DNS zurück übersetzt, um degenerierte Primer herzustellen. Die Primer hatten die folgenden Sequenzen (die Abkürzung I bezeichnet die Base Inosin):

PCR-Amplifikationen wurden entsprechend dem Stand der Technik nach den Angaben des Herstellers (PCR Kit von Qiagen, Hilden) durchgeführt: Eine 50 µl PCR Reaktion enthielt 100 ng chromosomaler T. versicolor DNS (isoliert wie im 2. Beispiel beschrieben), den vom Hersteller bereitgestellten Puffer und darüberhinaus 1,25 U Taq Polymerase, 1,25 mM MgCl₂, je 0,2 mM der vier dNTPs (dATP, dCTP, cGTP, dTTP) und jeweils 100 pmol der Primer A und B. Die weiteren Bedingungen zur spezifischen Amplifikation des gewünschten PCR-Produkts waren: 4 min bei 94°C, gefolgt von 10 Zyklen von 1 min bei 94°C, 1 min bei 45°C und 1 min bei 65°C sowie 30 Zyklen von 1 min bei 94°C, 1 min bei 50°C und 1 min bei 72°C. Es wurde ein PCR-Produkt von ca. 140 bp erhalten. Das PCR-Produkt wurde durch Agarose Gelelektrophorese gereinigt, in den pCR-Script Vektor (Klonierkit von Stratagene, Heidelberg) kloniert und in E. coli transformiert. Aus der Anzucht transformierter E. coli wurde das Plasmid isoliert. Eine DNS-Sequenzanalyse vom 5'- und 3'-Ende bestätigte, daß es sich bei dem klonierten DNS-Fragment um das Fragment eines pyrF-Gens handelte.

Zur Vorbereitung der DNS-Sonde für das Screening von pyrF-Genen wurde das pyrF-spezifische PCR-Fragment durch Behandlung mit Not I und Eco RI ausgeschnitten, über Agarose Elektrophorese isoliert und mit dem nicht-radioaktiven "Gene Images" Detektionskit der Fa. Amersham , Braunschweig markiert.

### 2. Beispiel

### Herstellung einer chromosomalen Genbank aus Trametes versicolor.

Es wurde der Stamm Trametes versicolor TV-1 (hinterlegt bei der DSMZ Deutschen Sammlung von Mikroorganismen und Zellkulturen GmbH, D-38124 Braunschweig unter der Nummer DSM 11523) verwendet. Mycel von Trametes versicolor wurde zuerst durch Anzucht für 7 Tage bei 28°C auf Malzagarplatten (3 % Malz Extrakt, 0,3 % Pepton aus Sojabohnenmehl, 1,5 % Agar-Agar, pH 5,0) gewonnen. Von den Malzagarplatten wurden drei Stücke ausgestanzt und damit 100 ml steriles Malzextrakt Medium (3 % Malz Extrakt, 0,3 % Pepton aus Sojabohnenmehl, pH 5,0) in 500 ml Erlenmeyerkolben angeimpft. Die Kultur wurde unter Schütteln bei 100 rpm für 7 Tage bei 28°C inkubiert. Die auf diese Weise hergestellte Mycelsuspension wurde über eine Porzellannutsche abgesaugt und mit 0,9 % Saline gewaschen. 1 g Mycel von T. versicolor wurden in Gegenwart von flüssigem Stickstoff mit Mörser und Pistill zu einem feinen Pulver zerrieben. Das Pulver wurde in einem sterilen Probengefäß aufgenommen und sofort mit 5 ml Extraktionslösung (0,1 M Tris-HCl, pH 8.0, 0,1 M EDTA, 0,25 M NaCl, 0,6 mg/ml Proteinase K) und 0,5 ml einer 10 % (w/v) Natriumlauroylsarcosinlösung versetzt. Nach Incubation bei 50°C für mindestens 2 h wurde das Gemisch mit 0,85 ml 5 M NaCl und 0,7 ml einer 10 % (w/v) CTAB-Lösung in 0,7 M NaCl versetzt und 30 min bei 65°C inkubiert. Nach Zugabe von 7 ml eines Chloroform-Isoamylalkoholgemisches (24:1) wurde der Ansatz ausgeschüttelt, die beiden Phasen durch Zentrifugation getrennt, die wässrige Phase entfernt und chromosomale DNS durch Zugabe von 0,6 Volumenanteilen Isopropanol gefällt. Die weitere Reinigung der gefällten DNS erfolgte anschließend über eine Säule (Qiagen Genomic Tip). Auf diese Weise konnten aus 16 g Mycel 0,5 mg chromosomale DNS isoliert werden.

Zur Herstellung der chromosomalen Genbank wurden 90 µg chromosomale DNS von Trametes versicolor TV-1 in einem Partialverdau mit Sau 3A geschnitten und durch Agarose Gelelektrophorese aufgetrennt. Die chromosomalen DNS-Fragmente wurden im Größenbereich von 5 - 20 kb und größer 20 kb isoliert und jeweils in mit Bam HI vorgeschnittene Lambda-Phagen kloniert ("Lambda Zap® Express" Klonierungssystem von Stratagene). Von der 5 - 20 kb DNS-Fraktion wurden 4 x 10⁴ Phagen/µg Vektor-DNS und von der DNS-Fraktion größer 20 kb wurden 5 x 10⁴Phagen/µg Vektor-DNS erhalten. Die Phagen wurden durch Infektion des E.coli Stammes XL-1 Blue MRF' amplifiziert.

### 3. Beispiel

### Isolierung des pyrF-Gens.

Es wurde die im 2. Beispiel beschriebene chromosomale Genbank von Trametes versicolor TV-1 verwendet. Das Screening nach dem genomischen pyrF-Gen wurde nach dem Stand der Technik durchgeführt. In einer ersten Screeningrunde wurden Zellen von E. coli XL-1 Blue MRF' auf 10 Petrieschalen zuerst kultiviert und dann mit 50000 Phagen der chromosomalen Genbank (5 - 20 kb Fraktion, siehe 2. Beispiel) pro Petrieschale infiziert. Nach Inkubation bei 37°C über Nacht wurden die neu gebildeten Phagen auf Nylonfilter (Stratagene) transferiert. Die Filter wurden dann entsprechend den Empfehlungen des Herstellers mit der nicht-radioaktiv markierten pyrF-spezifischen Sonde (siehe 1. Beispiel) hybridisiert. Die Hybridisierungstemperatur betrug 60°C. Positive Klone wurden gepickt und durch Wiederholung des Screeningverfahrens gereinigt. Nach drei Runden der Vereinzelung wurden bei dem Screening 6 stark hybridisierende Phagenklone isoliert, die nach einer Vorschrift des Herstellers (Stratagene) durch *"in vivo* excision" in den pBK CMV Vektor (Stratagene) umkloniert wurden. Analyse der Klone durch Verdau mit Restriktionsendonucleasen und PCR zeigte, daß es sich bei allen Klonen um pyrF-Gene handelte. Nach Sequenzanalyse von drei Klonen wurde vom längsten der pyrF-Klone ca. 3,4 kb Sequenzinformation ermittelt. Dieser pyrF-Klon erhielt die Bezeichnung pyrF61 (SEQ ID NO: 1). Der Klon pyrF61 enthielt Sequenzinformation für das pyrF-Strukturgen (kodierender Bereich, SEQ ID NO: 1, bp 1133 - 1877). Der kodierende Sequenzbereich enthielt zusätzlich ein Intron (SEQ ID NO: 1, bp 1226 - 1286), das nicht in Aminosäuresequenz translatiert wird. Das entsprechende pyrF cDNS-Gen ist in SEQ ID NO: 2 angegeben. Das in Klon pyrF61 enthaltene pyrF-Strukturgen, ohne die Intronsequenz, kodiert für ein Protein mit der in SEQ ID NO: 3 angegebenen Aminosäuresequenz.

Daneben enthielt Klon pyrF61 auch Sequenzinformation in der Region 5' vor dem ATG-Startkodon (Promotorbereich, SEQ ID No: 1, bp 1 - 1132) sowie Sequenzinformation in der Region 3' nach dem Stopkodon (Terminatorbereich, SEQ ID No: 1, bp 1878 - 3448). Es handelt sich hierbei um neue genetische Regulationselemente für Trametes versicolor, die für die Herstellung von Expressionsvektoren zur Genexpression in filamentösen Pilzen aus der Klasse der Basidiomyceten verwendet werden können.

### 4. Beispiel

### Funktionelle Verknüpfung des Trametes versicolor GAPDH-Promotors mit dem pyrF-Gen aus Trametes versicolor

### A: Klonieren des pyrF-Gens in den pBluescript Vektor

Für die weitere Bearbeitung wurde das pyrF-Gen aus pyrF61 in den Vektor pBluescript umkloniert. Dazu wurde das pyrF-Gen als 1,6 kb Sac I - Spe I Fragment aus dem im 3. Beispiel erhaltenen Klon pyrF61 isoliert und in den mit Sac I und Spe I vorgeschnittenen pBluescript Vektor subkloniert. Das dabei entstandene 4,6 kb Plasmid wurde pPyrF1 genannt.

### B: Einbau eines Linkers in pPyrF1 für die funktionelle Verknüpfung des ATG Translationsstartkodons des pyrF-Gens mit dem GAPDH-Promotor

Vektor pPyrF1 wurde mit Sac I geschnitten, der linearisierte, 4,6 kb große Vektor durch Agarose Gelelektrophorese isoliert und durch Behandlung mit alkalischer Phosphatase dephosphoryliert. Der auf diese Weise vorbereitete Vektor wurde mit den Linkeroligonukleotiden PyF-1 (SEQ ID NO:6) und PyF-2 (SEQ ID NO:7) ligiert. PyF-1 und PyF-2 hatten die folgende Sequenz:

In PyF-1 und PyF-2 ist die Schnittstelle für die Restriktionsendonuklease BspLU11 I unterstrichen, die für die funktionelle Verknüpfung mit dem GAPDH-Promotor aus T. versicolor verwendet werden kann.

Ligationsansätze von Sac I geschnittenem pPyrF1 mit den Linkeroligos PyF-1 und PyF-2 wurden in E. coli Top 10F'-Zellen transformiert. Positive Klone enthielten eine neu eingeführte BspLU11 I Schnittstelle (neben zwei bereits in pPyrF1 enthaltenen). Die richtige Orientierung des eingebauten Linkers, bei der am Start-ATG Kodon des pyrF-Gens eine BspLU11 I Schnittstelle eingeführt worden war, wurde durch DNS-Sequenzanalyse bestimmt. Der auf diese Weise hergestellte Vektor (ca. 4,5 kb Größe) erhielt die Bezeichnung pPyrF2.

### C: Einbau des T. versicolor GAPDH-Promotors in den pUC19 Vektor

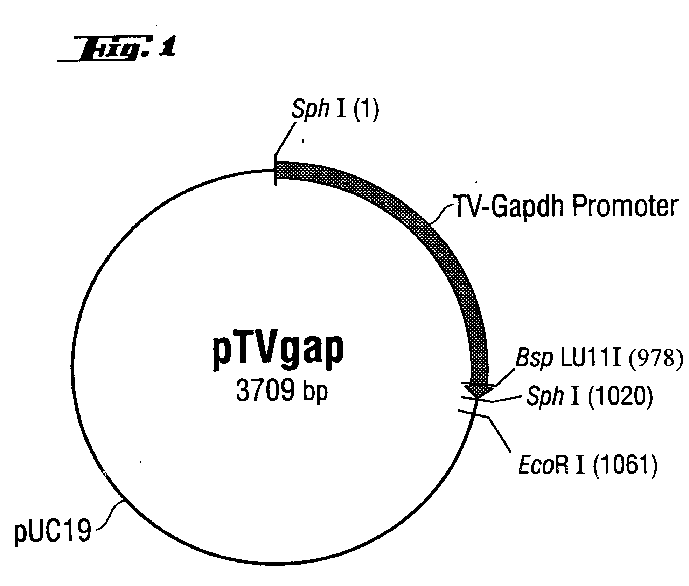

Die DNS-Sequenz des Promotors für das T. versicolor GAPDH-Gen ist in DE-A-19814853, SEQ ID NO:3, bp 1 - 1542 offenbart. Ein ca. 1 kb großes Promotorfragment des GAPDH-Gens wurde als Sph I Fragment isoliert und in einen pUC19 Vektor kloniert. Nach Analyse durch Doppelverdau mit den Restriktionsendonukleasen Eco RI (im Polylinker von pUC19 enthalten) und BspLU11 I (im GAPDH-Promoterfragment enthalten) wurde ein Klon ausgewählt, in dem die BspLU11 I Schnittstelle der Eco RI Schnittstelle benachbart war. Der auf diese Weise hergestellte 3,7 kb große Vektor erhielt die Bezeichnung pTVgap (Fig. 1).

Aus dem für die Herstellung von pTVgap verwendeten pUC19 Vektor war vorher eine singuläre BspLU11 I Schnittstelle entfernt worden, die bei der weiteren Vektorkonstruktion störend gewesen wäre. Dies erfolgte, indem der pUC19 Vektor mit BspLU11 I geschnitten und der dadurch linearisierte Vektor mit Klenow DNA-Polymerase behandelt wurde. Dadurch wurden die nach dem BspLU11 I Verdau versetzten Enden des pUC19 Vektors aufgefüllt. Anschließende Ligation und Transformation von E. coli Top 10F' ergab Klone, die einen modifizierten pUC19 Vektor ohne BspLU11 I Schnittstelle enthielten.

### D: Funktionelle Verknüpfung des GAPDH-Promotors mit dem pyrF-Gen

Der Vektor pTVgap wurde mit BspLU11 I und Eco RI geschnitten, das dabei entstandene 3,7 kb große Vektorfragment durch Agarose Gelelektrophorese isoliert und durch Behandlung mit alkalischer Phosphatase dephosphoryliert.

Aus dem Vektor pPyrF2 wurde das pyrF Gen als 1,6 kb großes BspLU11 I - Eco RI Fragment isoliert. Dazu wurde pPyrF2 zuerst partial mit BspLU11 I geschnitten und das 4,6 kb große, linearisierte Vektorfragment durch Agarose Gelelektrophorese isoliert. Das isolierte 4,6 kb Fragment wurde anschließend mit Eco RI nachgeschnitten. Dabei entstand das gewünschte 1,6 kb pyrF Genfragment, das durch Agarose Gelelektrophorese isoliert wurde.

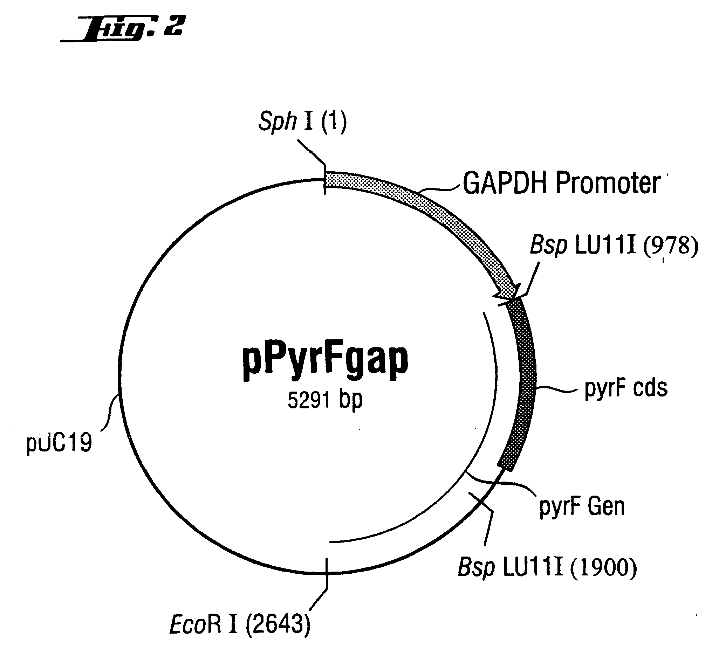

Das 3,7 kb große BspLU11 I - Eco RI Vektorfragment aus pTVgap und das 1,6 kb großes BspLU11 I - Eco RI Fragment aus pPyrF2 wurden ligiert und mit dem Ligationsansatz E. coli Top 10F' Zellen transformiert. Klone, bei denen das pyrF Gen über die BspLU11 I Schnittstelle funktionell mit dem GAPDH-Promotor verknüpft worden war, wurden durch Restriktionsanalyse identifiziert. Durch DNS-Sequenzierung wurde die korrekte Verknüpfung des GAPDH-Promotors mit dem Start-ATG Kodon des pyrF Gens bestätigt. Der korrekte Klon hatte eine Größe von 5,3 kb und erhielt die Bezeichnung pPyrFgap (Fig. 2).

### 5. Beispiel:

### Herstellung von Trametes Protoplasten und Regenerierung von Pilzkolonien

Für die Gewinnung von Protoplasten verwendet wurden die dikaryotischen Stämme Trametes versicolor TV-1, Trametes versicolor 38070 (erhältlich von American Type Culture Collection, Rockville, MD 20852 USA) und der monokaryotische Stamm Trametes versicolor F2 100 (hinterlegt bei der DSMZ Deutschen Sammlung von Mikroorganismen und Zellkulturen GmbH, D-38124 Braunschweig unter der Nummer DSM 11972). Mycel von Trametes versicolor wurde zuerst durch Anzucht für 7 Tage bei 28°C auf Malzagarplatten (3 % Malz Extrakt, 0,3 % Pepton aus Sojabohnenmehl, 1,5 % Agar-Agar, pH 5,0) gewonnen. Von den Malzagarplatten wurden drei Stücke ausgestanzt und damit 100 ml steriles Malzextrakt Medium (3 % Malz Extrakt, 0,3 % Pepton aus Sojabohnenmehl, pH 5,0) in 500 ml Erlenmeyerkolben, bzw. 125 ml des sterilen Mediums in 162 cm² Zellkulturgefäßen, angeimpft. Die Kultur wurde ohne Schütteln für 7 Tage bei 28°C inkubiert, bis die Kulturflüssigkeit dicht mit einer Mycelmatte bewachsen war. Die Kulturflüssigkeit wurde dekantiert und frisches Medium zugegeben (30 ml für das Mycel einer 100 ml Anzucht). Das Mycel wurde mit einem Ultra Turrax (9500 rpm, 4 min) homogenisiert und unter Schütteln bei 100 rpm für weitere 18 h bei 28°C inkubiert.

Die auf diese Weise hergestellte Mycelsuspension wurde durch Zentrifugation für 5 min bei 1500 rpm (2000 x g) geerntet und das dabei erhaltene Mycel dreimal durch suspendieren mit 0,1 M MgSO₄, 0,6 M Saccharose, 0,1 M Phosphat, pH 5,8 (OMT-Medium) und anschließendes Zentrifugieren gewaschen. Das isolierte Mycel wurde gewogen und bis zur Behandlung mit lytischem Enzym bei. 4°C aufbewahrt.

Protoplasten wurden folgendermaßen hergestellt: In einem sterilen Erlenmeyerkolben wurde Mycelium eines Kolbens in 15 ml einer frisch hergestellten und sterilfiltrierten Lösung des lytischen Enzymgemisches Novozym 234 (3 mg/ml, Novo Nordisk, Bagsvaerd, Dänemark) in OMT-Medium suspendiert. Das in der Enzymlösung resuspendierte Mycelium wurde bei geringer Drehzahl (80 rpm) auf einem Schüttelinkubator (Infors) für 1 bis 3 h bei 30°C inkubiert. Während der Inkubation wurde die Bildung der Protoplasten im Mikroskop beobachtet. Frei bewegliche Protoplasten waren üblicherweise nach 1 h zu sehen. Der Endpunkt der Protoplastierung wurde durch visuelle Inspektion im Mikroskop bestimmt und die Protoplasten durch Filtration über Glaswolle in einem Glasfilter von restlichem Mycelium abgetrennt. Die Glaswolle wurde sorgfältig mit eisgekühltem OMT-Medium gewaschen. Protoplasten wurden durch Zentrifugation der Suspension in einem sterilen Probengefäß isoliert (2000 rpm; 2500 x g, 4°C, 10 min). Die weitere Bearbeitung der Zellen erfolgte bei 4°C. Das Protoplastenpellet wurde durch suspendieren in OMT-Medium gewaschen und durch Zentrifugation reisoliert. Bei Bedarf wurde der Waschschritt wiederholt. Die Konzentration von Protoplasten wurde unter dem Mikroskop in einer Zählkammer bestimmt. Die Protoplastensuspension wurde für Experimente zur Protoplastenregenerierung oder für Transformationen auf eine Konzentration von 1 x 10⁸ Protoplasten/ml eingestellt.

Für Regenerierungsexperimente wurden von der Protoplastensuspension Verdünnungsreihen hergestellt und auf Agarplatten ausplattiert, die 1,5 % Malzextrakt, 0,1 % Trypticase Pepton, 2 % Glucose, 1,5 % Agar und zur osmotischen Stabilisierung 0,4 M Mannitol enthielten. Auf diese Weise wurde der Anteil an überlebensfähigen Zellen bestimmt und getestet, ob die erhaltenen Protoplasten zu mycelartigem Wachstum regeneriert werden konnten. Auf die gleiche Weise wurde auf osmotisch nicht stabilisierten Platten (ohne Mannitol) der Anteil an osmotisch stabilen Zellen (z.B. Mycelfragmente) bestimmt. Nach Inkubation bei 28°C für 7 Tage wurden die erhaltenen Kolonien gezählt. Der Anteil überlebensfähiger Zellen aus einer Reihe von Protoplastenpräparationen betrug ca. 0,5 %. Diese Ergebnisse zeigen, daß von Trametes versicolor überlebensfähige und regenerierbare Protoplasten für Transformationsexperimente hergestellt werden können.

### 6. Beispiel

### Isolierung von Uridin-auxotrophen Mutanten von Trametes versicolor

Uridin-auxotrophe Mutanten von Trametes versicolor mit einem Gendefekt im Pyrimidin Stoffwechsel (pyr-Mutanten) wurden in Anlehnung des in Boeke et al., Methods Enzymol. (1987) 154, 164 - 175 beschriebenen Verfahrens isoliert. Als selektives Agens wurde die genotoxische Substanz 5-Fluor-Orotsäure (FOA) verwendet. Mutagenese von Trametes versicolor Protoplasten erfolgte durch UV-Behandlung.

### A: UV-Mutagenese:

Für die Mutagenese verwendet wurde der im 5. Beispiel beschriebene monokaryontische Stamm Trametes versicolor F2 100. Protoplasten dieses Stammes wurden hergestellt wie im 5. Beispiel beschrieben.

Für die Mutagenese wurde als Quelle für UV-Licht ein BioRad UV-Linker (BioRad, München, Leistung 5,8 W/cm², Abstand von der UV-Quelle 16 cm) verwendet. Die Zahl der für die Mutagenese verwendeten Protoplasten betrug 8 x 10⁹. Protoplasten von Trametes versicolor wurden in einer Petrischale vorgelegt und für verschieden lange Zeitabschnitte mit UV-Licht bestrahlt. Dabei stellte sich heraus, daß unter den beschriebenen Bedingungen eine Bestrahlung für 60 sec. optimal für die nachfolgende Selektion auxotropher Mutanten war.

### B: Selektion von Uridin auxotrophen Mutanten:

Für die Selektion von Uridin auxotrophen Mutanten wurde folgendes Minimalmedium (MM) verwendet:

| | |
|---|---|
| Glucose | 20 g/l |
| Agar | 15 g/l |
| Kaliumdihydrogenphosphat | 1 g/l |
| Magnesiumsulfat | 0,5 g/l |
| Dinatriumhydrogenphosphat | 0,1 g/l |
| Adenin | 27,5 mg/l |
| DL-Phenylalanin | 0,15 g/l |
| L-Asparagin | 2,5 g/l |
| Thiamin | 0,48 mg/l |
| Calciumchlorid | 10 mg/l |
| Eisensulfat | 10 mg/l |
| Kupfersulfat | 2 mg/l |
| Zinksulfat | 1 mg/l |
| Mangansulfat | 1 mg/l |
| pH 5,0, mit Schwefelsäure eingestellt. | |

Für die osmotische Stabilisierung von Protoplasten wurde das MM mit 0, 6 M Saccharose supplementiert (MMS). Für Flüssiganzuchten wurde das MM ohne Agar hergestellt.

Zu Beginn wurde das MMS mit verschiedenen Konzentrationen FOA sowie 10 mM Uridin supplementiert, um die Wachstumseigenschaften auf selektivem Medium für verschiedene Trametesstämme zu charakterisieren. Es stellte sich heraus, daß MMS mit 1,5 mg/ml FOA und 10 mM Uridin (selektives MMS) das Wachstum der untersuchten Trametesstämme vollständig unterdrückte.

Platten mit selektivem MMS wurden mit UV-mutagenisierten Protoplasten (in Abschnitt A beschrieben) beimpft und 21 Tage bei 28°C inkubiert. Im Gegensatz zu nicht mutagenisierten Protoplasten wurde das Wachstum von 35 Kolonien beobachtet. Diese potentiellen pyr-defizienten Mutanten wurden, um den Uridin auxotrophen Phänotyp näher zu charakterisieren, auf MM-Platten, MM-Platten mit 10 mM Uridin und selektiven MM-Platten ausgebracht und das Wachstum zum Ausgangsstamm F2 100 verglichen. Dabei zeigten von den 35 gepickten Kolonien 13 Trametesmutanten eindeutig einen pyr-defizienten Phänotyp. Dies ist beispielhaft in der Tabelle 1 für den Wildtypstamm und drei Mutanten dargestellt.

**Tabelle 1**

| **Wachstum von Trametes versicolor Mutanten auf verschiedenen MM** | | | |
|---|---|---|---|
| Stamm | MM | MM + 10 mM Uridin | MM + 10 mM Uridin + 1,5 mg/ml FOA |
| F2 100 | + | + | - |
| F2 100C2-1 | - | + | + |
| F2 100C2-8 | - | + | + |
| F2 100C4-13 | - | + | + |

### C: Identifizierung von pyrF-Mutanten

Die Mutagenese mit FOA kann entweder zu Mutanten im gewünschten pyrF Gen (Orotsäure-Phosphoribosyltransferase) oder im pyrG Gen (Orotidin-5'-Phosphatdecarboxylase) führen. pyrG Mutanten und pyrF Mutanten wurden differenziert durch Transformation mit dem pyrF-Gen aus Trametes versicolor, dessen Isolierung im 3. Beispiel beschrieben wurde(Plasmid pyrF61). Parallel dazu wurden Uridin-auxotrophe T. versicolor Stämme auch mit dem Plasmid pPyrFgap transformiert (Herstellung siehe 4. Beispiel). Die Transformation von Trametes versicolor wird im 7. Beispiel beschrieben.

Bei 6 der 13 isolierten pyr-defizienten Mutanten konnten nach Transformation mit den Plasmiden pyrF61 und pPyrFgap Kolonien auf MM beobachtet werden. Dies deutet darauf hin, daß diese sechs Mutanten defizient im pyrF Gen waren. Die drei Stämme F2 100C2-1, F2 100C2-8 und F2 100C4-13 ließen sich wiederholt mit der höchsten Häufigkeit transformieren und wurden für die weiteren Untersuchungen verwendet. Ein Vergleich der Plasmide pyrF61 und pPyrFgap bezüglich der Transformationshäufigkeit ergab keine signifikanten Unterschiede, sbdaß der pyrF-Promotor ausreichend für die Isolierung von Transformanten war.

Bei dem im 2. Beispiel beschriebenen pyrF-Gen handelt es sich um ein neues Selektionsmarkergen für die Transformation von Trametes versicolor. Bei den Stämmen Trametes versicolor F2 100C2-1, F2 100C2-8 und F2 100C4-13 handelt es sich um die ersten bisher beschriebenen pyrF-defizienten Stämme von Trametes versicolor. Diese pyrF-defizienten Stämme können als Wirtsorganismen dienen für die Transformation von Trametes versicolor und sind somit neue wertvolle Wirtsorganismen für die Proteinexpression und Proteinsekretion in filamentösen Pilzen aus der Klasse der Basidiomyceten. Die Verwendung des Stammes F2 100C2-1 zu diesem Zweck wird in den folgenden Beispielen beschrieben.

### 7. Beispiel

### Transformation von pyrF-defizienten Trametes versicolor Stämmen mit dem pyrF-Gen aus Trametes versicolor

### A: Isolierung von Transformanten

Protoplasten von T. versicolor F2 100C2-1 wurden nach dem im 5. Beispiel beschriebenen Verfahren hergestellt. Dabei war das Anzuchtsmedium für den auxotrophen Stamm mit 10 mM Uridin supplementiert worden. Es wurde mit dem Vektor pyrF61 (beschrieben im 3. Beispiel), bzw. pPyrFgap (beschrieben im 4. Beispiel) transformiert.

Wie im 5. Beispiel beschrieben, wurden Protoplasten von Trametes versicolor F2 100C2-1 hergestellt und in einer Endkonzentration von 10⁸/ml suspendiert. In Inkubationsgefäßen von 12 ml Volumen wurden 0,1 ml Aliquots der Protoplasten mit je 10 µg DNS des betreffenden Plasmids gemischt und 30 min auf Eis inkubiert. Danach wurde langsam und unter wiederholtem mischen 1,25 ml einer PEG4000 Lösung zum Transformationsansatz gegeben. Nach weiteren 20 min Inkubation bei Raumtemperatur wurden die Reaktionsgefäße mit dem im 5. Beispiel beschriebenen OMT-Medium aufgefüllt, gemischt und 10 min bei 4°C bei 2000 x g zentrifugiert. Die Pellets wurden resuspendiert und auf osmotisch stabilisierten MMS-Platten ohne Uridin (beschrieben im 6. Beispiel) ausplattiert. Die Platten wurden bei 28°C für 14 Tage inkubiert und auf Wachstum von Kolonien überprüft. Bei verschiedenen Experimenten wurden Transformationsraten von 0,5 - 3 Transformanten/µg Plasmid DNS erzielt.

### B: Reinigung der Transformanten

Mycel der erhaltenen Transformanten wurde gepickt und durch Ausplattieren auf frische Platten mit MM gereinigt. Dabei wurde das Inokulum punktförmig in der Mitte der Platte aufgebracht. Nach Inkubation für ca. 7 Tage bei 28°C konnte radiales Mycelwachstum beobachtet werden. Dieser Reinigungsvorgang wurde wiederholt, wobei das Mycel für das Inokulum vom Rand der ersten Reinigungsplatte entnommen wurde. MM-Platten wurden dann erneut mit Inokulum der zweiten Reinigungsplatte inokuliert und bei 28°C inkubiert, bis die Platten vollständig mit Mycel bewachsen waren.

### C: Analyse der Transformanten

Transformanten von Trametes versicolor wurden mittels Southernblot Analyse auf die Integration des Plasmids PyrF61, untersucht. Dazu wurde von verschiedenen Transformanten und als Kontrolle dem pyrF-defizienten Stamm F2 100C2-1 Mycel in Flüssigkultur hergestellt (siehe 2. Beispiel, Malzextrakt-medium, für F2 100C2-1 mit 10 mM Uridin versetzt). Von dem isolierten Mycel wurde chromosomale DNS isoliert wie im 2. Beispiel beschrieben.

Von den untersuchten Transformanten und dem nicht transformierten, Uridin-auxotrophen Ausgangsstamm F2 100C2-1 wurden je 3 µg der chromosomalen DNS, von pyrF61 100 ng des Plasmids mit Nco I geschnitten, anschließend durch Agarose Gelelektrophorese getrennt und auf Nylonfilter (Qiagen) geblottet. Als DNS-Sonde wurde Nco I geschnittenes Plasmid pyrF61 verwendet, nicht-radioaktiv markiert wie im 1. Beispiel beschrieben. Mit dieser DNS-Sonde konnten sowohl das pyrF Gen sowie die Vektoranteile aus dem jeweiligen Plasmid nachgewiesen werden.

Die Hybridisierungstemperatur für die auf Nylonfilter geblottete DNS mit der nicht-radioaktiv markierten DNS-Sonde betrug 60°C. Ansonsten wurden die in der Fachliteratur beschriebenen Bedingungen für Southernblots eingehalten. Southernblots wurden durch Autoradiographie ausgewertet. Neben anderen Fragmenten konnten zwei, vom pBK CMV-Vektoranteil von pyrF61 stammende Nco I Fragmente mit einer Länge von 0,7 kb, bzw. 1,9 kb detektiert werden. Diese beiden Fragmente konnten nur in den Transformanten, nicht jedoch in dem Uridin-auxotrophen Stamm F2 100C2-1 nachgewiesen werden. Dieses Ergebnis bestätigt, daß bei der Transformation des Uridin-auxotrophen Stammes Trametes versicolor F2 100C2-1 das Plasmid pyrF61 in das Genom integriert worden war und zur produktiven Expression des Selektionsmarkergens pyrF führte, wodurch die Uridin Auxotrophie dieses Stammes komplementiert wurde.

### 8. Beispiel

### Verwendung des pyrF-Gens zur Herstellung Laccaseüberproduzierender Trametes versicolor Stämme

### A. Transformation von T. versicolor

Protoplasten von T. versicolor wurden nach dem im 5. Beispiel beschriebenen Verfahren'hergestellt. Zur Transformation verwendet wurde der im 3. Beispiel beschriebene Vektor pyrF61 sowie der Laccase Expressionsvektor pLac3gap. Die beiden Vektoren wurden in Co-Transformationsexperimenten verwendet, wo das Selektionsmarkergen und das zu exprimierende Gen auf getrennten Plasmiden enthalten sind. Die Herstellung von pLac3gap ist in in DE-A-19814853, 6. Beispiel, offenbart. In pLac3gap ist das Gen für die Laccase III aus T. versicolor funktionell mit dem dem GAPDH Promotor aus T. versicolor verknüpft.

Wie im 7. Beispiel beschrieben, wurden Protoplasten des pyrF-defizienten Stammes Trametes versicolor F2 100C2-1 hergestellt und in einer Endkonzentration von 10⁸/ml suspendiert. In Inkubationsgefäßen von 12 ml Volumen wurden 0,1 ml Aliquots der Protoplasten mit je 10 µg DNS der Plasmide pLac3gap und pyrF61 gemischt und 30 min auf Eis inkubiert. Danach wurde langsam und unter wiederholtem mischen 1,25 ml einer PEG4000 Lösung zum Transformationsansatz gegeben. Nach weiteren 20 min Inkubation bei Raumtemperatur wurden die Reaktionsgefäße mit dem im 5. Beispiel beschriebenen OMT-Medium aufgefüllt, gemischt und 10 min bei 4°C bei 2000 x g zentrifugiert. Die Pellets wurden resuspendiert und auf osmotisch stabilisiertem MM ohne Uridin (beschrieben im 6. Beispiel) ausplattiert. Die Platten wurden bei 28°C für 14 Tage inkubiert und auf Wachstum von Kolonien überprüft. Bei verschiedenen Experimenten wurden Transformationsraten von 0,5 - 3 Transformanten/µg DNS des Selektionsmarkerplasmids pyrF61 erzielt.

Die erhaltenen Transformanten wurden gepickt und wie im 7. Beispiel beschrieben zweimal durch ausplattieren auf frischen Platten mit MM-Selektionsmedium ohne Uridin gereinigt. Selektivplatten wurden dann erneut mit Inokulum der zweiten Reinigungsplatte inokuliert und nachdem die Platten vollständig mit Mycel bewachsen waren, wurde die Laccaseproduktion in Schüttelkolbenanzuchten überprüft.

### B: Anzucht im Schüttelkolben

Für die Anzucht im Schüttelkolben wurden 2 cm² Mycel aus einer voll bewachsenen Platte ausgestochen, steril zerkleinert und damit eine Vorkultur von 50 ml (in einem 250 ml Erlenmeyerkolben) Malzextrakt-Medium (siehe 1. Beispiel) beimpft. Die Vorkultur wurde 6 Tage bei 28°C unter Schütteln bei 120 rpm inkubiert. Am sechsten Tag wurde die Vorkultur mit einem Ultra Furrax für 30 sec bei 9500 rpm homogenisiert und damit 250 ml Hauptkulturmedium (Zusammensetzung siehe MM im 6. Beispiel) in einem 11 Erlenmeyerkolben beimpft. Die Hauptkultur wurde dann wieder bei 28°C unter Schütteln bei 120 rpm inkubiert. Die Laccaseproduktion wurde ab dem zweiten Tag der Anzucht täglich gemessen. Laccaseaktivität wurde photometrisch mit dem Substrat ABTS (2,2'-Azino-bis(3-Ethyl-Benzthiazolin-6-Sulfonsäure)) bei 420 nm gemessen. (Extinktionskoeffizient von ABTS bei 420 nm ε₄₂₀: 3, 6 x 10⁴ [1 x Mol⁻¹ x cm⁻¹]). Dabei entsprach 1 U Laccaseaktivität dem Umsatz von 1 µmol ABTS/min bei 37°C und einem pH von 4,5. Das Maximum der Laccaseproduktion in Schüttelkolbenanzuchten wurde 10 - 14 Tage nach Beginn der Hauptkultur erreicht. Tabelle 2 zeigt einen Vergleich verschiedener Transformanten mit dem nicht transformierten Ausgangsstamm Trametes versicolor F2 100. Für den nicht transformierten Stamm F2 100 wurde darüberhinaus die Laccaseproduktion nach Induktion mit dem in der Literatur beschriebenen Induktor 2,5-Xylidin (Yaver et al., Applied and Environmental Microbiology (1996) 62, 834 - 841) bestimmt. Wie aus Tabelle 2 zu ersehen, ist die Laccaseproduktion im Schüttelkolben bei den besten Transformanten des Stammes F2 100 gegenüber dem nichttransformierten Ausgangsstamm um den Faktor 14 (ohne Induktion), bzw. um den Faktor 6 (mit Induktion) erhöht.

**Tabelle 2**

| Stamm Trametes versicolor | Maximale Laccaseproduktion (U/ml) |
|---|---|
| F2 100 | 4,60' |
| F2 100/Xylidin* | 11,20 |
| TV L3F-4 | 15,20 |
| TV L3F-7 ' | 42,50 |
| TV L3F-9 | 17,60 |
| TV L3F-14 | 51,50 |
| TV L3F-21 | 33,90 |
| TV L3F-29 | 64,80 |
| TV L3F-35 | 35,10 |
| TV L3F-38 | 13,80 |
| TV L3F-51 | 56,70 |

| | |
|---|---|
| * Die Induktion erfolgte drei Tage nach Beginn der Hauptkultur durch Zugabe von 2,5-Xylidin (1,5 mM Endkonzentration). | |

## Patentansprüche

1. Expressionssystem umfaßend einen Wirtsstamm ausgewählt aus den filamentösen Pilzen aus der Klasse der Basidiomyceten mit einem genetischen Defekt im Stoffwechselmetabolismus aufgrund dessen der für das Wachstum essentielle Stoffwechselmetabolit Uridin nicht mehr synthetisiert werden kann und der Wirtsstamm auf Minimalmedien ohne Zusatz dieses Stoffwechselmetaboliten nicht mehr zum Wachstum befähigt ist sowie einen Expressionsvektor enthaltend ein Selektionsmarkergen, welches den auxotrophen Gendefekt des Wirtsstammes komplementiert, **dadurch gekennzeichnet, dass** der Wirtsstamm als genetischen Defekt im Stoffwechselmetabolismus einen Defekt im pyrF Gen besitzt, und das Selektionsmarkergen, das pyrF Gen aus einem Pilz der Klasse der Basidiomyceten ist.

2. Expressionssystem gemäß Anspruch 1, **dadurch gekennzeichnet, dass** der Wirtsstamm ausgewählt ist aus den Gattungen Trametes, Coriolus und Polyporus.

3. Verfahren zur Herstellung eines Proteins, **dadurch gekennzeichnet, dass** ein Expressionssystem gemäß Anspruch 1 oder 2 enthaltend ein das Protein kodierendes Gen zur Proteinproduktion eingesetzt wird oder
dass ein Mikroorganismus der einen Expressionsvektor umfassend eine DNS-Sequenz, die für ein Protein mit Enzymaktivität der Orotsäure-Phosphoribosyltransferase (pyrF-Aktivität)kodiert und eine DNS-Sequenz SEQ ID NO:1 ab Position 1133 bis einschließlich Position 1877 umfaßt oder eine DNS-Sequenz SEQ ID NO:2 ab Position 1 bis einschließlich Position 684 umfaßt oder eine DNS-Sequenz mit einer Sequenzhomologie größer als 70 % zu den genannten Bereichen der DNS-Sequenz SEQ ID NO:1, oder SEQ ID NO:2 umfasst enthaltend ein das Protein kodierendes Gen kultiviert wird und das Protein aus der Kultur gewonnen wird.

4. Verfahren gemäß Anspruch 3 **dadurch gekennzeichnet, dass** das Protein Laccase ist.

## Claims

1. Expression system comprising a host strain selected from the filamentous fungi from the class Basidiomycetes having a genetic defect in biotransformation metabolism, on the basis of which the biotransformation metabolite uridine which is essential for growth can no longer be synthesized, and the host strain is no longer able to grow on minimal media without addition of this biotransformation metabolite, and an expression vector comprising a selection marker gene which complements the auxotrophic gene defect of the host strain, **characterized in that** the host strain has as genetic defect in biotransformation metabolism a defect in the pyrF gene, and the selection marker gene is the pyrF gene from a fungus of the class Basidiomycetes.

2. Expression system according to claim 1, **characterized in that** the host strain is selected from the genera Trametes, Coriolus and Polyporus.

3. Process for producing a protein, **characterized in that** an expression system according to claim 1 or 2 comprising a gene encoding the protein is employed for protein production, or **in that** a microorganism which comprises an expression vector comprising a DNA sequence which codes for a protein having the enzymatic activity of orotate phosphoribosyltransferase (pyrF activity), and a DNA sequence SEQ ID NO: 1 from position 1133 up to and including position 1877, or comprises a DNA sequence SEQ ID NO: 2 from position 1 up to and including position 684, or comprises a DNA sequence having a sequence homology of more than 70% with the said regions of the DNA sequence SEQ ID NO: 1, or SEQ ID NO: 2, comprising a gene encoding the protein is cultivated, and the protein is obtained from the culture.

4. Process according to claim 3, **characterized in that** the protein is laccase.

## Revendications

1. Système d'expression comprenant une souche hôte choisie parmi les champignons filamenteux de la classe des basidiomycètes avec un défaut génétique dans le métabolisme en raison duquel l'uridine, un métabolite essentiel pour la croissance, ne peut plus être synthétisé et la souche hôte n'est plus apte à la croissance sur des milieux minimaux sans addition de ce métabolite ainsi qu'un vecteur d'expression contenant un gène d'un marqueur de sélection qui est complémentaire du défaut de gène auxotrophe de la souche hôte, **caractérisé en ce que** la souche hôte présente comme défaut génétique dans le métabolisme un défaut dans le gène pyrF et le gène du marqueur de sélection est le gène pyrF d'un champignon de la classe des basidiomycètes.

2. Système d'expression selon la revendication 1, **caractérisé en ce que** la souche hôte est choisie parmi les genres Trametes, Coriolus et Polyporus.

3. Procédé pour la production d'une protéine **caractérisé en ce qu'**on utilise un système d'expression selon la revendication 1 ou 2, contenant un gène codant pour la protéine, pour la production de la protéine ou **en ce qu'**on cultive un microorganisme qui contient un vecteur d'expression comprenant une séquence d'ADN codant pour une protéine présentant l'activité enzymatique de l'acide orotique-phosphoribosyltransférase (activité pyrF) et comprenant une séquence d'ADN SEQ ID NO:1 à partir de la position 1133 jusqu'à la position 1877 comprise ou une séquence d'ADN SEQ ID NO:2 à partir de la position 1 jusqu'à la position 684 comprise ou une séquence d'ADN présentant une homologie de séquence supérieure à 70% avec les zones mentionnées de la séquence d'ADN SEQ ID NO:1 ou SEQ ID NO:2, contenant un gène codant pour la protéine et on produit la protéine à partir de la culture.

4. Procédé selon la revendication 3, **caractérisé en ce que** la protéine est une laccase.


### SEQUENZPROTOKOLL

<110> Consortium für elektrochemische Industrie GmbH
<120> pyrF Gen und seine Verwendung
<130> Co9904
<140>
   <141>
<160> 7
<170> PatentIn Vers. 2.0
<210> 1
   <211> 3448
   <212> Dann
   <213> Trametes versicolor
<220>
   <221> gene
   <222> (1133)..(1877)
<220>
   <221> promoter
   <222> (1)..(1132)
<220>
   <221> 3'UTR
   <222> (1878)..(3448)
<220>
   <221> intron
   <222> (1226) ... (1286)
<400> 1
   gatctcgagt aggatggaga acggtataac gatgccagag atgataggtg tccagcggta 60
   gttgggaacg aggctgtcta ggtcggcgtt cttgtcgtcg gtcggccaga ggtacgattt 120
   gaggcgagag tatagagata tccctaggag tgtgacgtca tcggacgagt taccctccgc 180
   ggtatgttgt gtactgtcca ccttctccgg gtcagacggg tgtgatgtac tgcgtccggg 240
   ttggagcgtc aggaaaagcg cagacaggct gaagagtccc attccgccgc agaaggtgtg 300
   cgagggggaa agtgccagtg tagcagtgag gcgtgcctac gatatgagac ggacatggtc 360
   agtatctatg cccggtcaag gtcgccgcac agacctcact gtaacctcat ggcaatgtcg 420
   cggatgcaca aagcaggtag agatgttcaa atggggcacg gagcgggtcg tccggagcgc 480
   tctccctcgg ctctttgcaa ggcagctggc ggatgtttgg tcagttgagg tactgcatcc 540
   cttgcaatag cgaaaacagc tcaccagacg tgagtatatg ctgtatacgg gagaaggaag 600
   cggaacaccg tgagtggaag agatgaagtg gttatgaata catcccggtg gaggttgagt 660
   ctaacagcgt cggatctcgc tgcgttccgg agcagaggcc cggtacgagc gccggtgtct 720
   gctcgtgttc cggcacgccg tatgctcgta aatcaccttt agaaaacttg aataagtgag 780
   agaagatacg aaacgtcagt ctgcacctat ggagatatgt aaaaatcgca aaaacatagc 840
   gttgacgcta taaaaaagaa aaggacaaaa tgaccaccgc aggggtcgaa cctgcaatct 900
   cctgatccct aggtttgaag gttcatcacc tcaattcgta gtcagacgcg atgccatttc 960
   gccaggcggc cgttagaaac gaaactacta cgtttaaacc cgggtataac acagcctagt 1020
   attccgtgcg ggccgcgccg ccgataagct tgttttcgtg aactgtcttc cccctcctgc 1080
   atctcgattc tcgacctcca tcgccgcgac gatcccttcc ttcccactca ccatgtcgct 1140
   cgaaaaatac cagacagagc tcatcgagca cggcatgacc gccggtgcgc tcaagttcgg 1200
   gaccttcacc ctcaaatcag gccggtccgt cccctcccta ggctgcgcgc cgctctcccc 1260
   gtgaacgctc cctcaccccg cgcaggacct cgccctactt cttcaacgcc ggcctgctcg 1320
   cgtccgggcc cgtgctcgac acgctgtgct ccgcgtacgc cgcgacgatc gcgcgcgcgc 1380
   tcaaggcgtc gcccgggctg cccgcgttcg acgtgctctt cgggcccgcg tacaagggca 1440
   tcccgttcgc ggcggggacc gcgctgctgc tgcaccgcga ccacggcatc accgtcgggt 1500
   tcgcgtacga ccgcaaggag gcgaaggatc atggggaggg cgggatactt gtgggcgcgc 1560
   cggtgagggg caagcgcgtg ctggtgctgg acgacgtcgc gacggcgggc acggcgatcc 1620
   gccaggcgat tgagactgtg acgaaggagg ggggcgaggt cgttggcgcg gtgttgatgc 1680
   tcgatcggca ggaggtgggc aaggagggga agagcacgct tgcggaggtg gaggcgctgt 1740
   tgggcgggaa gggacgtgtg ccgacgatcc tgaggatgaa ggacctcatg aagtggttgc 1800
   aggagcacgg ccggacggag gagcttgcga agatgcaaga gtactgggag cagtacggcg 1860
   cgaaggaaag cgaatgagaa gacacgaagg cagttgtgta ctaggtgagt aacaccacgc 1920
   tacatcgatc catccactaa acccatgcag atgaagaccc actgtacaat ttctcggtac 1980
   ctgtcacgtt gaacgcaaag agccgaagat gtgagagtac acatgccatt catcccgata 2040
   tatagcacaa gaacatgtag taatagaacc tgcagaaaca caaagcatga tcagcaagac 2100
   tccatgggca ctgagttatg atgaactaac cgctatcacc aaaaacaccg ctcttattcg 2160
   cccaaccgac gacccgaacc ccagttatat cctcccacac cgctcgcagc agcagcagca 2220
   gcagcctgct ccctgaccct ccgtgggggc acaacatgca cgcccccacc gacattcgca 2280
   acgcccccga ccccacccgt cgcgccccca ctagcagact ccccgaacca cccgcgcagc 2340
   cacgccggcg ccgtacctgc agccctgagc gagccggtgt caaagacagt ccaccaccag 2400
   aggtggccga cgacagcccc agagatgctg atggcagcgg cgccggggcc gcccatgagg 2460
   aggtccatgc cgacgagcat gtagggaagg tagatgacgg ggaaggtgat gagcccgaag 2520
   aaggatgtct gggacccagg tggggcgagc cgggaggaga cgtaggtgag cgcgaggagg 2580
   agcgcgcggg tgtgcacaaa ggtgccgagg ggaatgttga ggccctggtg tgaggcgggt 2640
   tagcgcaaag gtcagaggcg ggatgatact attggacgta cgaggatagc aagtcctgcg 2700
   agcgagagct gccatgcgta gtctgaagag cggcggggga agtgtgtctc ttctagctca 2760
   ttggaatttc gactagtttc aagtgtacgg tcctcagtat catcatgtat tgcaacagtg 2820
   tcatacgcac tagagcatcg caaggtcgaa gatgaagttg atccccgagc ctataaagac 2880
   aaggtcagca ccgacatggc atgtagtcag acaagattga gtacgcactt cccaagaaga 2940
   agctcgtaaa cactctccag atctacatta agacgtgagt atcgcatacc ttctcagtgc 3000
   ctgacttatc tttcatccaa ctacagagac agaaacccac ctcaaacttc tgcgtaacga 3060
   actccttcac aaagacgacc ttgtatattg gcaagatttg caggagcact ggcaaggtga 3120
   cggcgagaga ggaggcgcat agaaaccgag tgactggagg gattttgcga atctcatcca 3180
   tgaaagacat cttgaggaga ctggaggtga gtagagcgat agaagtacag caggcagagc 3240
   agagacgacg gcagaatgtg gggaagaaca agcaggagga ggagtagagt gattttgaag 3300
   taatgaaaag tggcgcaacc taatgcaaag tgtatgaggg acatccgtgg acataaagta 3360
   ttccgcacct cgggcaagac attcaatctc agtaatgcac ttcactttcg gagttcaact 3420
   tcaaactcga ctttgaaact tgagatcc 3448
<210> 2
   <211> 684
   <212> Dann
   <213> Trametes versicolor
<220>
   <221> CDS
   <222> (1)..(684)
<400> 2
   atg tcg ctc gaa aaa tac cag aca gag ctc atc gag cac ggc atg acc    48
   Met Ser Leu Glu Lys Tyr Gln Thr Glu Leu Ile Glu His Gly Met Thr
    1               5                  10                  15
   gcc ggt gcg ctc aag ttc ggg acc ttc acc ctc aaa tca ggc cgg acc    96
   Ala Gly Ala Leu Lys Phe Gly Thr Phe Thr Leu Lys Ser Gly Arg Thr
               20                  25                  30
   tcg ccc tac ttc ttc aac gcc ggc ctg ctc gcg tcc ggg ccc gtg ctc   144
   Ser Pro Tyr Phe Phe Asn Ala Gly Leu Leu Ala Ser Gly Pro Val Leu
           35                  40                  45
   gac acg ctg tgc tcc gcg tac gcc gcg acg atc gcg cgc gcg ctc aag   192
   Asp Thr Leu Cys Ser Ala Tyr Ala Ala Thr Ile Ala Arg Ala Leu Lys
       50                  55                  60
   gcg tcg ccc ggg ctg ccc gcg ttc gac gtg ctc ttc ggg ccc gcg tac     240
   Ala Ser Pro Gly Leu Pro Ala Phe Asp Val Leu Phe Gly Pro Ala Tyr
    65                  70                  75                  80
   aag ggc atc ccg ttc gcg gcg ggg acc gcg ctg ctg ctg cac cgc gac     288
   Lys Gly Ile Pro Phe Ala Ala Gly Thr Ala Leu Leu Leu His Arg Asp
                    85                  90                  95
   cac ggc atc acc gtc ggg ttc gcg tac gac cgc aag gag gcg aag gat     336
   His Gly Ile Thr Val Gly Phe Ala Tyr Asp Arg Lys Glu Ala Lys Asp
               100                 105                 110
   cat ggg gag ggc ggg ata ctt gtg ggc gcg ccg gtg agg ggc aag cgc     384
   His Gly Glu Gly Gly Ile Leu Val Gly Ala Pro Val Arg Gly Lys Arg
           115                 120                 125
   gtg ctg gtg ctg gac gac gtc gcg acg gcg ggc acg gcg atc cgc cag     432
   Val Leu Val Leu Asp Asp Val Ala Thr Ala Gly Thr Ala Ile Arg Gln
       130                 135                 140
   gcg att gag act gtg acg aag gag ggg ggc gag gtc gtt ggc gcg gtg     480
   Ala Ile Glu Thr Val Thr Lys Glu Gly Gly Glu Val Val Gly Ala Val
   145                 150                 155                 160
   ttg atg ctc gat cgg cag gag gtg ggc aag gag ggg aag agc acg ctt     528
   Leu Met Leu Asp Arg Gln Glu Val Gly Lys Glu Gly Lys Ser Thr Leu
                   165                 170                 175
   gcg gag gtg gag gcg ctg ttg ggc ggg aag gga cgt gtg ccg acg atc     576
   Ala Glu Val Glu Ala Leu Leu Gly Gly Lys Gly Arg Val Pro Thr Ile
               180                 185                 190
   ctg agg atg aag gac ctc atg aag tgg ttg cag gag cac ggc cgg acg     624
   Leu Arg Met Lys Asp Leu Met Lys Trp Leu Gln Glu His Gly Arg Thr
           195                 200                 205
   gag gag ctt gcg aag atg caa gag tac tgg gag cag tac ggc gcg aag     672
   Glu Glu Leu Ala Lys Met Gln Glu Tyr Trp Glu Gln Tyr Gly Ala Lys
       210                 215                 220
   gaa agc gaa tga                                                     684
   Glu Ser Glu
   225
<210> 3
   <211> 227
   <212> PRT
   <213> Trametes versicolor
<400> 3
   Met Ser Leu Glu Lys Tyr Gln Thr Glu Leu Ile Glu His Gly Met Thr
   1               5                   10                  15
   Ala Gly Ala Leu Lys Phe Gly Thr Phe Thr Leu Lys Ser Gly Arg Thr
               20                  25                  30
   Ser Pro Tyr Phe Phe Asn Ala Gly Leu Leu Ala Ser Gly Pro Val Leu
           35                  40                  45
   Asp Thr Leu Cys Ser Ala Tyr Ala Ala Thr Ile Ala Arg Ala Leu Lys
       50                  55                  60
   Ala Ser Pro Gly Leu Pro Ala Phe Asp Val Leu Phe Gly Pro Ala Tyr
   65                  70                  75                  80
   Lys Gly Ile Pro Phe Ala Ala Gly Thr Ala Leu Leu Leu His Arg Asp
                   85                  90                  95
   His Gly Ile Thr Val Gly Phe Ala Tyr Asp Arg Lys Glu Ala Lys Asp
               100                 105                 110
   His Gly Glu Gly Gly Ile Leu Val Gly Ala Pro Val Arg Gly Lys Arg
           115                 120                 125
   Val Leu Val Leu Asp Asp Val Ala Thr Ala Gly Thr Ala Ile Arg Gln
       130                 135                 140
   Ala Ile Glu Thr Val Thr Lys Glu Gly Gly Glu Val Val Gly Ala Val
   145                 150                 155                 160
   Leu Met Leu Asp Arg Gln Glu Val Gly Lys Glu Gly Lys Ser Thr Leu
                   165                 170                 175
   Ala Glu Val Glu Ala Leu Leu Gly Gly Lys Gly Arg Val Pro Thr Ile
               180                 185                 190
   Leu Arg Met Lys Asp Leu Met Lys Trp Leu Gln Glu His Gly Arg Thr
           195                 200                 205
   Glu Glu Leu Ala Lys Met Gln Glu Tyr Trp Glu Gln Tyr Gly Ala Lys
       210                 215                 220
   Glu Ser Glu
   225
<210> 4
   <211> 26
   <212> Dann
   <213> Artificial Sequence
<220>
   <223> Die Beschreibung von Knstliche Sequenz:PrimerA
<220>
   <221> primer_bind
   <222> (1)..(26)
<220>
   <221> primer_bind
   <222> (1)..(26)
   <223> n = i
<400> 4
   ttyggnccng cntayaargg nathcc 26
<210> 5
   <211> 23
   <212> Dann
   <213> Artificial Sequence
<220>
   <223> Die Beschreibung von Knstliche Sequenz:PrimerB
<220>
   <221> primer_bind
   <222> Complement ((1)..(23))
<220>
   <221> primer_bind
   <222> Complement ((1)..(23))
   <223> n = i
<400> 5
   ttnccnccyt cnccrtgrtc ytt 23
<210> 6
   <211> 35
   <212> Dann
   <213> Artificial Sequence
<220>
   <223> Die Beschreibung von Knstliche Sequenz:PyF-1
<220>
   <221> misc_feature
   <222> (1)..(35)
<400> 6
   ctagacatgt cgctcgaaaa ataccagaca gagct 35
<210> 7
   <211> 35
   <212> Dann
   <213> Artificial Sequence
<220>
   <223> Die Beschreibung von Knstliche Sequenz:PyF-2
<220>
   <221> misc_feature
   <222> Complement((1)..(35))
<400> 7
   ctgtctggta tttttcgagc gacatgtcta gagct                                  35